# EUROPÄISCHE PATENTSCHRIFT

(11) **EP 1 744 686 B1**
(45) Veröffentlichungstag und Bekanntmachung des Hinweises auf die Patenterteilung: **06.04.2016**
(21) Anmeldenummer: 05714957.7
(22) Anmeldetag: 10.02.2005
(51) Int. Cl.: A61B 5/00, A61B 17/56

(54) **BAUTEIL UND VERFAHREN ZUM ZUSAMMENBAU EINER IMPLANTATANORDNUNG**
COMPONENT AND METHOD FOR ASSEMBLING AN IMPLANT ARRANGEMENT
COMPOSANT ET PROCÉDÉ DE MONTAGE D'UN SYSTÈME D'IMPLANT

(30) Priorität: 10.02.2004 DE 102004006501
(43) Veröffentlichungstag der Anmeldung: 24.01.2007
(73) Patentinhaber: Synthes GmbH, 4436 Oberdorf (CH)
(72) Erfinder: DUDA, Georg, 12209 Berlin (DE); HELLER, Markus, 10115 Berlin (DE)
(74) Vertreter: Höhfeld, Jochen
(86) Internationale Anmeldenummer: PCT/DE2005/000233
(87) Internationale Veröffentlichungsnummer: WO 2005/074821

(56) Entgegenhaltungen:
- WO-A-01/19239
- WO-A-01/49173
- WO-A-03/092514
- DE-A1- 10 048 375
- US-A- 4 186 749
- US-A- 5 725 578
- US-A- 5 769 893
- US-A- 5 964 993
- US-A- 6 034 296
- US-A1- 2002 151 770
- US-B1- 6 464 687

## Beschreibung

Die Erfindung liegt auf dem Gebiet der Technologie von Implantaten.

Implantate werden sowohl beim Menschen als auch bei Tieren genutzt, um Funktionen des Knochenskeletts zu unterstützen oder sogar Teile des Skeletts zu ersetzen. Zu den Implantaten, die Funktionen des Skeletts unterstützen und deshalb auch als Stützimplantate bezeichnet werden können, gehören Platten, Schienen oder winkelstabile Systeme aus einem festen Material, beispielsweise Edelstahl oder Titan, die an Skelettknochen befestigt werden, um deren Stützfunktion zu übernehmen. Derartige Stützimplantate werden beispielsweise im Zusammenhang mit Knochenbrüchen verwendet, um Knochenteile während des Heilungsprozesses relativ zueinander zu fixieren. Hierbei werden die Implantate beispielsweise mit Hilfe von Schrauben an den Knochen befestigt. Um bei Knochenfrakturen oder in der plastischen Chirurgie die Knochenflächen zusammenzufügen, werden spezielle Implantate verwendet, zum Beispiel Platten oder winkelstabile Systeme. Zur Fixierung der Knochen werden die Knochenfragmente insbesondere mittels Schrauben oder Bolzen mit den Platten verbunden. Um eine Anpassung der Osteosynthese an die jeweilige Patientensituation zu erlauben, sind die Platten mit mehreren Öffnungen zur Aufnahme der Schrauben oder Bolzen versehen.

Die Heilung, beispielsweise die Heilung eines Knochenbruchs, ist ein biologischer Prozeß, dessen Verlauf von zahlreichen biologischen aber auch bio-mechanischen Umgebungsfaktoren abhängt. Es gibt Vorschläge, die Heilung durch systemische oder lokale Applikation stimulierender Substanzen zu unterstützen. Die Freisetzung entsprechender Substanzen im zeitlichen Verlauf und die Auswirkungen auf relevante bio-physikalische und/oder bio-chemische Parameter am Ort des Interesses kann heute nur unzureichend beurteilt werden. Unabhängig von erfolgter/nicht-erfolgter Unterstützung wird derzeit der Fortschritt des Heilungsprozesses anhand von Verfahren beurteilt, die mit schwerwiegenden Nachteilen behaftet sind.

Die Auswertung von Röntgenbildern ist das Standardverfahren zur Dokumentation des Heilungsfortschrittes bei der Knochenbruchheilung. Dieses Verfahren ist jedoch mit einer Strahlenbelastung für den Patienten verbunden und somit ein invasives Verfahren, bei dem darüber hinaus eine objektivierbare Dokumentation des Heilungsforschrittes nur beschränkt möglich ist. Die Entscheidung, wann der Heilungsprozeß soweit abgeschlossen ist, daß beispielsweise ein temporär eingebrachtes Implantat wieder entfernt werden kann, wird daher wesentlich von der Erfahrung des Chirurgen bestimmt. Verfahren zur Beurteilung des Heilungsfortschrittes auf Basis von Computer-Tomographie sind aufgrund der Strahlungsbelastung ebenfalls invasive Verfahren. Bei liegendem Implantat ist die Bewertung in der Regel schwierig, aber auch zeit- und ressourcenintensiv und mit sehr hohen Kosten verbunden, so daß eine routinemäßige Anwendung nicht möglich ist. Ähnliches gilt für MR basierte Verfahren, die zeitintensiv, bei liegendem Implantat nicht immer anwendbar oder von begrenzter Aussagekraft, kostenintensiv und nicht in großer Zahl einsetzbar sind. Bei speziellen Verfahren zur Stabilisierung von Frakturen, zum Beispiel mittels sogenannter "Fixateur externe", sind nicht-invasive Verfahren anwendbar, welche die Veränderung in der Relativbewegung der Knochenfragmente mit Hilfe optischer Messungen berührungslos dokumentieren (G. N. Duda, B. Bartmeyer, S. Sporrer, W. R. Taylor, M. Raschke, N. D. Haas; "Does partial weight bearing unload a healing bone in external ring fixation?"; Langenbecks Arch Surg., Oktober 2003, 388(5), 298-304.) und somit den Verlauf der Heilung darstellen können. Für Stabilisierungsverfahren, bei welchen die Implantate, beispielsweise Platten, winkelstabile "Fixateur interne", unter der Haut liegen und die Befestigungselemente einer optischen Messung nicht zugänglich sind, ist das zuvor beschriebene berührungslose Verfahren nicht anwendbar.

US 6,034,296 beschreibt ein Sensor-System zur Befestigung an einem Implantat. Das Sensor-System ist mit einem Druck-Meßsystem ausgestattet, um die im Implantat auftretenden mechanischen Spannungen im alltäglichen Gebrauch zu messen. Dazu ist das Sensor-System mit Hilfe von Schrauben oder mittels geschweißter Verbindungen an dem Implantat befestigt. Außerdem umfaßt das Sensor-System eine Telemetreieinheit, um die gemessenen Werte an einen externen Empfänger zu senden.

DE 198 58 889 A1 offenbart ein Implantat zur Fixierung von Knochen. Als eine Ausführungsform wird vorgeschlagen, an dem Implantat einen Sensor für die Ermittlung der auf das Implantat einwirkenden Kräfte sowie eine Telemetrieeinheit für die Übermittlung der Meßergebnisse an eine externe Einheit anzuordnen.

Die WO 01/49173 A1 offenbart ein satzungsgemäßes Bauteil in Form einer Sonde zur in-viva-Messung von Drücken im oder am Knochen. Die Sonde umfasst ein Grundbauteil, ein in dem Grundbauteil angeordneter Drucksensor zum Erfassen einer Messgröße und zum Erzeugen von Messdaten für die erfasste Messgröße, eine in dem Grundbauteil angeordnete Telemetrieeinrichtung zum Senden und/oder Empfangen von Daten und einen elektrischen Leiter zwischen dem Drucksensor und der Telemetrieeinrichtung zum Übertragen von Daten zwischen dem Drucksensor und der Telemetrieeinnchtung. In einer Variante weist dieses Grundbauteil Bohrungen für Knochenschrauben zur Befestigung der Sonde am Knochen auf. In einer anderen Variante wird die Sonde direkt in den Knochen eingeschraubt.

Die US 4,186,749 A offenbart einen Detektor zur Messung von intrakraniellen Drücken die Außenseite der Hirnhaut (dura mater). Der Detektor weist einen Drurkmessruhler und eine Telemetrieeinrichtung auf, die in einem mehrteiligen Grundkörper angeordnet sind, wobei ein zylindrisches Gehäuse des Grundkörpers entlang einem Abschnitt ein Außengewinde aufweist Zur Fixierung des Detektors wird zuerst ein Loch in der Schädeldecke erstellt und anschließend ein Kragen mittels Schrauben am Schädel fixiert, der eine Gewindeöffnung aufweist, in die eine Hülse einschraubbar ist, in welche dann der Detektor eingeschraubt wird.

Die US 2002/0141770 A1 offenbart eine Vorrichtung mit einem Grundbautell, in dem eine Sensoreinrichtung und eine Telemetrieeinrichtung vorgesehen sind. Die vom Sensor erfassten Werte können zur Steuerung eines vom Sensor baulich getrennten und zu diesem beabstandeten Wirkstoffabgabesystems verwendet werden. Das Grundbauteil kann mit einem Außengewinde versehen sein, so dass das Grundbauteil in ein zuvor in den Schädel gebohrtes Loch einschraubbar ist.

Die US 6,464,687 B1 offenbart eine implantierbare Vorrichtung zur Abgabe eines Wirkstoffs im Körper mit mehreren kleinen Kugeln, von geringem Durchmesser. In der Ausführungsform gern. Fig. 7 umfasst eine derartige Vorrichtung mehrere Sensor-Kugeln, eine Prozessor-Kugel, eine Timer-Kugel, welche die Sensor-Kugeln (702) zum Messen veranlasst, welche dann die Messwerte an eine Transmitter-Kugel senden, wobei die Transmitter-Kugel (708) mit einem separaten, beabstandet von dieser Vorrichtung angeordneten Reservoir und mit einer Empfangseinrichtung außerhalb des Körpers (600) kommuniziert. Über eine Schraube ist die Vorrichtung mit dem umgebenden Gewebe verbindbar.

Die WO 01/19239 A1 offenbart eine implantiere Vorrichtung, beispielsweise einen Stent, welcher in Gefäßen implantiert werden kann, um den Durchfluss des Gefäßes sicherzustellen. Die Vorrichtung weist Sensoren für die Datenmessung und eine Telemetrieeinrichtung auf, wobei Mittel zur Arzneimittelabgabe auf Basis der ermittelten Werte der Sensoren vorgesehen sind. Erfasst beispielsweise ein Sensor die Gegenwart unerwünschter Zellansammlungen innerhalb des Stents, kann von außen die Freigabe von Protease gesteuert werden, welche in einem Behälter im Stent vorgesehen ist.

Aufgabe der Erfindung ist es, ein verbessertes Bauteil anzugeben, daß in Verbindung mit einem Implantat nutzbar ist, so daß die Erfassung medizinisch relevanter Meßgrößen erleichtert wird. Darüber hinaus soll die Montage des Bauteils möglichst einfach sein.

Diese Aufgabe wird erfindungsgemäß durch ein System mit einem Bauteil, sowie einem Stützimplantat oder einem Ersatzimplantat, sowie durch ein Bauteil nach den unabhängigen Ansprüchen gelöst.

Die Erfindung umfaßt den Gedanken, ein Bauteil zum Anordnen an einem Implantat zur Verfügung zu stellen, wobei das Bauteil die Merkmale des unabhängigen Anspruch 1 aufweist.

Hierdurch ist eine Möglichkeit geschaften, einen medizinischen Wirkstoff zusammen mit dem Implantat zu implantieren, wobei der Wirkstoff in dem Aufnehmeraum untergebracht ist.

Auf diese Weise wird ein Bauteil zur Verfügung gestellt, das auf einfache Weise an dem zu implantierenden Implantat montiert werden kann. Die Montage des Grundbauteils mit der mindestens einen Sensoreinrichtung und der Telemetrieeinrichtung an dem Implantat hat den Vorteil, daß hierdurch in räumlicher Nähe zu dem Implantat, welches seinerseits im Körper des Lebewesens an einem Ort plaziert ist, an dem ein zu beobachtender Prozeß, beispielsweise ein Heilungsprozeß, abläuft, mit Hilfe der Sensoreinrichtung Meßdaten erfaßt werden können, die dann nach einer Übertragung von der Sensoreinrichtung an die Telemetrieeinrichtung von außerhalb des Körpers des Lebewesens von der Telemetrieeinrichtung mittels Auslesen von Daten abgerufen ("passive Ausführung", zum Beispiel mittels einer Transponder-Technik) oder von der Telemetrieeinrichtung gesendet ("aktive Ausführung") und in einer Auswerteeinrichtung ausgewertet werden können. Die mit Hilfe der vorgeschlagenen konstruktiven Ausgestaltung des Bauteils ermöglichte Integration von Sensoreinrichtung und Telemetrieeinrichtung an dem Implantat unterstützt eine räumlich möglichst nahe Meßwerterfassung zu dem Ort, an dem Änderungen im Verlauf des Heilungsprozesses beobachtet werden können. So ermöglicht diese Konstruktion des Bauteils nach Einbau des Bauteils in das Implantat beispielsweise die Überwachung von Frakturheilungen. Sensoreinrichtungen, die als solche dem Fachmann bekannt sind, können auf diese Art und Weise mit geringem Aufwand an einem Implantat angeordnet werden. Die mit Hilfe der Sensoreinrichtung ausgeführte Messung betrifft beispielsweise eine pH-Wert-Messung, eine Sauerstoffpartialdruck-Messung, eine Druckmessung (Flüssigkeitsdruck), eine Beschleunigungsmessung und/oder eine Proteinkonzentrationsmessung.

Eine zweckmäßige Ausgestaltung der Erfindung kann vorsehen, daß das Bauteil in einer Implantatausnehmung angeordnet ist. Hierdurch wird die feste Lokalisierung des Bauteils an dem Implantat erleichtert.

Eine mit geringem Aufwand ausführbare Möglichkeit zum Montieren des Grundbauteils am Implantat ist bei einer bevorzugten Ausgestaltung der Erfindung dadurch geschaffen, daß die Montagemittel einen Montageabschnitt zum wenigstens teilweisen Einführen in die Implantatausnehmung umfassen. Das wenigstens teilweise Einführen des Montageabschnitts des Grundbauteils sichert eine stabile Halterung des Grundbauteils an dem Implantat.

Bei einer Ausgestaltung der Erfindung kann vorgesehen sein, daß im Bereich des Montageabschnitts ein Gewindeabschnitt zum Einschrauben des Grundbauteils in die Implantatausnehmung gebildet ist. Die mit Hilfe des Gewindeabschnitts ermöglichte Schraubbefestigung des Bauteils ermöglicht ein zuverlässiges Befestigen und Lösen des Bauteils an dem Implantat.

Ein Hindurchrutschen des Bauteils durch die Implantatausnehmung ist bei einer vorteilhaften Ausführungsform der Erfindung dadurch verhindert, daß das Grundbauteil im Längsschnitt einen im wesentlichen T-förmigen Querschnitt mit einem Kopfteil und einem Basisteil aufweist. Das im Vergleich zum Basisteil breitere Kopfteil verhindert, daß das Bauteil zu weit in die Implantatausnehmung hinein gelangt.

Bei einer Weiterbildung der Erfindung kann vorgesehen sein, daß die mindestens eine Sensoreinrichtung im Bereich eines Endabschnitts des Grundbauteils und die Telemetrieeinrichtung im Bereich eines gegenüberliegenden Endabschnitts des Grundbauteils angeordnet sind. Hierdurch wird erreicht, daß einerseits die mindestens eine Sensoreinrichtung möglichst nahe einem zu untersuchenden Ort angeordnet werden kann, an dem die Meßgröße erfaßt werden soll. Andererseits kann die Telemetrieeinrichtung bei dieser Ausgestaltung möglichst so angeordnet werden, daß ein störungsfreies Empfangen/Senden der Daten gewährleistet ist. Dieses wird bei einer weiteren Ausführungsform der Erfindung insbesondere dadurch unterstützt, daß die Telemetrieeinrichtung im wesentlichen in dem Kopfteil des Grundbauteils angeordnet ist. Das Kopfteil wird beim Anordnen des Bauteils an dem Implantat nicht in die Implantatausnehmung eingerührt, so daß es möglichst weitgehend freigelegt ist, was das ungestörte Senden/Empfangen von Daten erleichtert.

Zum kontrollierten Abgeben des Werkstoffs aus dem Aufnahmeraum durch die Öffnung im Rahmen einer Wirkstoffappllikation ist bei einer vorteilhaften Ausgestaltung der Erfindung eine Abgabeeinrichtung vorgesehen. Die Abgabeeinrichtung kann eine Pumpeinrichtung zum Pumpen einer Menge des Wickstoffs aus dem Aufnahmeraum durch die Öffnung umfassen. Darüber hinaus sieht eine bevorzugte Weiterbildung der Erfindung vor, daß die Abgabeeinrichtung einen Öffnungsmechanismus zum Öffnen/Schließen der Öffnung umfaßt. Auf diese Weise sind verschiedenen Ausführungsformen eines Bauteils gebildet, das dazu verwendet werden kann, sowohl Meßdaten zu erfassen und diese mit Hilfe der Telemetrieeinrichtung zu überrtagen als auch einem Wirkstoff an einem Ort in Implantat Nähe zur Verfügung zu stellen. Mikromechanismen zum Bilden der Abgabeeinrichtung, einer Pumpe und/oder des Öffnungsmechanismus sind dem Fachmann als solche beispielsweise aus dem Bereich der Mikrosystantechnik in medizinischen oder nicht-medizinischen Anwendungen bekannt.

Zweckmäßig ist bei einer Ausführungsform der Erfindung vorgesehen, daß die Abgabeeinrichtung über eine weitere Datenübertragungsverbindung zum Übertragen von Daten mit der Telemetrieeinrichtung verbunden ist. Hierdurch ist die Möglichkeit geschaffen, von außen elektronische Daten an die Abgabeeinrichtung zu übersenden und/oder elektronische Daren von der Abgabeeinrichtung über die Telemetrieeinrichtung zu empfangen. Hierdurch kann die Abgabe des Werkstoffes von außerhalb des Körpers, in welchem das Implantat mit dem Bauteil verwendet wird, kontrolliert werden.

Darüber hinaus kann eine Fortbildung der Erfindung eine Steuereinheit vorsehen, die mit der mindestens einen Sensoreinrichtung und der Abgabeeinrichtung verbunden ist, um die Erfassung der Meßdaten mit Hilfe der mindestens einen Sensoreinrichtung und die Abgabe des Wirkstoffs mit Hilfe der Abgabeeinrichtung gemeinsam zu steuern. Eine gemeinsame Steuerung bedeutet in diesem Zusammenhang im wesentlichen eine aufeinander abgestimmte Steuerung, so daß das Erfassen von Meßdaten und das Abgeben des Wirkstoffs beispielsweise in zeitlicher Hinsicht miteinander korreliert werden können.

Das beschriebene Bauteil mit der mindestens einen Sensoreinrichtung, der Telemetrieeinrichtung und den Montagemitteln am Grundbauteil kann sowohl in einer Implantatausnehmung eines Stützimplantats, insbesondere einer Platte, eines "Nagels" (Marknagel) oder einer Schiene aus einem Material mit hoher Steifigkeit, als auch in einer Implantatausnehmung eines Ersatzimplantats, insbesondere eines künstlichen Hüft-, Knie- oder Schultergelenkts, angeordnet werden. Hierbei ist der Montageaufwand minimiert, wenn die Implantatausnehmung eine beim Implantieren nutzbare Montageausnehmung zum Aufnehmen von Implantat-Befestigungsmitteln ist. Derartige Montageausnehmungen sind in der Regel an Implantaten vorgesehen, um das Implantat mit Hilfe von Schrauben oder ähnlichen Befestigungsmitteln zu befestigen. Die Anordnung des Bauteils in einer solchen Montageaufnahme, die für die Befestigung des Implantats redundant ist, vermeidet, daß das Implantat zusätzlich mechanisch bearbeitet werden muß, um eine Anordnung des Bauteils zu ermöglichen. Eine einfache aber hinsichtlich der mechanischen Befestigung sichere Anordnung des Bauteils ist bei einer Ausgestaltung der Erfindung dadurch gewährleistet, daß die Implantatausnehmung einen Innengewindeabschnitt aufweist, in welche das Bauteil eingeschraubt ist.

Die Erfindung wird im folgenden anhand von Ausführungsbeispielen unter Bezugnahme auf eine Zeichnung näher erläutert. Hierbei zeigen:
- Figur 1: eine Anordnung mit einem Knochen und einem hieran befestigten Implantat;
- Figur 2: eine Querschnittsdarstellung der Anordnung nach Figur 1 entlang einer Linie AA';
- Figur 3: ein Bauteil zum Montieren an einem Implantat mit einer Sensoreinrichtung und einer Telemetrieeinrichtung im Querschnitt;
- Figur 4: ein weiteres Bauteil zum Montieren an einem Implantat mit einer Sensoreinrichtung und einer Telemetrieeinrichtung im Querschnitt;
- Figur 5: eine schematische Darstellung eines Wirbelkörpers mit einem hieran befestigten Implantat; und
- Figur 6: ein anderes Bauteil zum Montieren an einem Implantat mit einer Sensoreinrichtung und einer Telemetrieeinrichtung im Querschnitt.

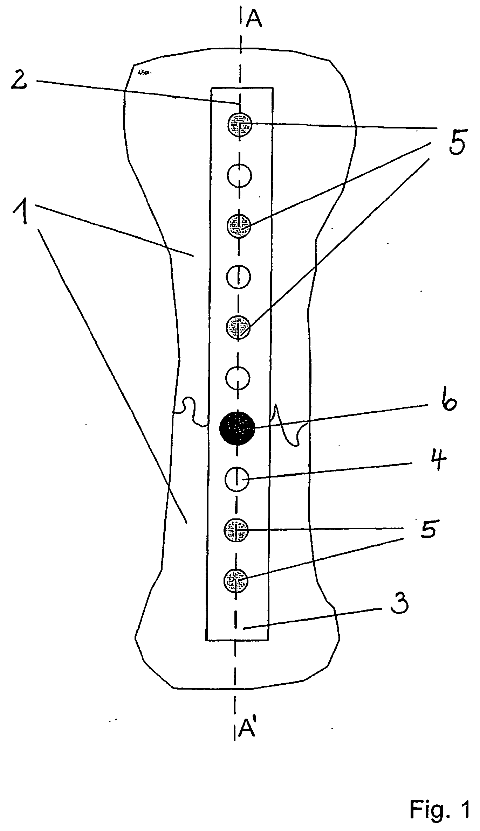

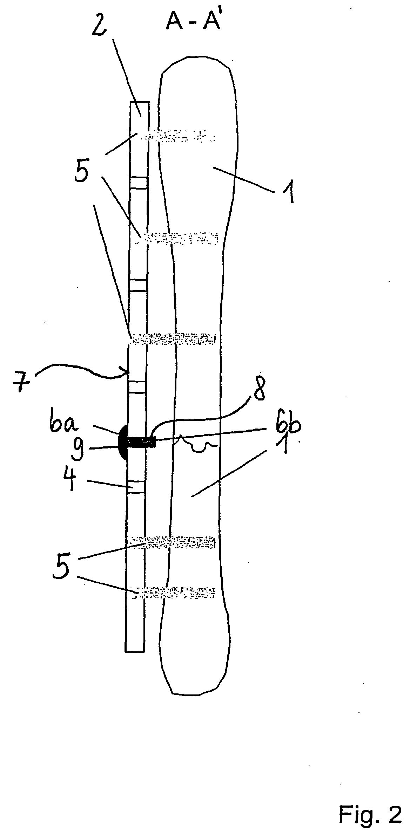

Figur 1 zeigt ein Knochenfragment 1 mit einem hieran befestigten Implantat 2, bei dem es sich um eine Schiene 3 mit mehreren Öffnungen 4 handelt, die als Durchgangsbohrungen ausgeführt sind. In einem Teil der Öffnungen 4 sind Schrauben 5 angeordnet, die zur Befestigung des Implantats 2 an dem Knochenfragment 1 dienen. Zu diesem Zweck sind die Schrauben 5 in das Knochenfragment 1 eingeschraubt, was sich deutlicher aus Figur 2 ergibt, welche einen Querschnitt durch das Knochenfragment 1 und das Implantat 2 nach Figur 1 entlang einer Linie AA' zeigt.

Ein Teil der zur Montage des Implantats 2 nutzbaren Öffnungen 4 in der Schiene 3 werden nicht zum Befestigen mittels der Schrauben 5 genutzt. In eine dieser nicht genutzten Öffnungen ist ein Bauteil 6 eingeführt. Das Bauteil 6 kann hierbei in die Schiene 3 eingesteckt oder eingeschraubt sein. Ein Kopfteil 6a des Bauteils 6 ist auf einer Außenfläche 7 der Schiene 3 angeordnet. Ein sich von dem Kopfteil 6a erstreckendes Basisteil 6b des Bauteils 6 erstreckt sich durch die Öffnung in der Schiene 3 hindurch, so daß ein Endabschnitt 8 des Basisteils 6b dem Knochenfragment 1 gegenüberliegend angeordnet ist. Ein gegenüberliegender Endabschnitt 9 im Bereich des Kopfteils 6a ist von dem Knochenfragment 1 entfernt und im wesentlichen frei auf der Außenfläche 7 der Schiene 3 plaziert.

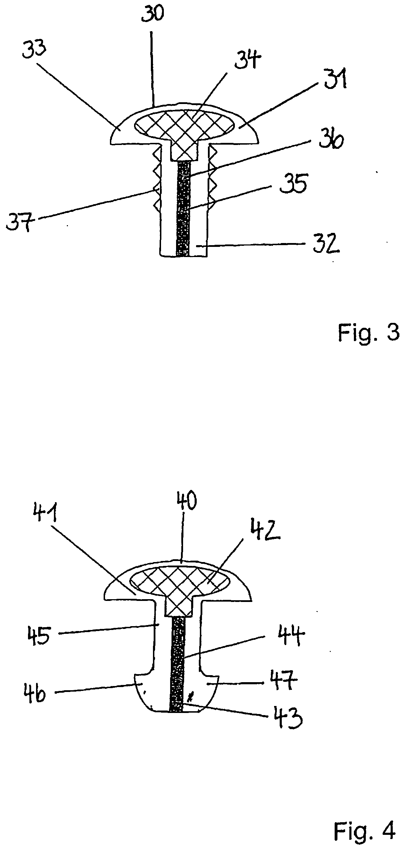

Ausführungsformen des Bauteils 6 sind in den Figuren 3 und 4 detaillierter dargestellt. Bei der Ausführungsfonm eines Bauteils 30 nach Figur 3 weist das Bauteil 30 im gezeigten Längsschnitt einen pilzförmigen Querschnitt mit einem Kopfteil 31 und einem Basisteil 32 auf. Das Bauteil 30 umfaßt ein Grundbauteil 33, in welchem eine Telemetrieeinrichtung 34 und eine Sensoreinrichtung 35 angeordnet sind. Die Sensoreinrichtung 35 und die Telemetrieeinrichtung 34 sind über eine Datenübernagungseinrichtung 36 miteinander verbunden. Mit Hilfe der Sensoreinrichtung 35 können Meßdaten für eine oder mehrere physikalische Meßgröße erfaßt werden, beispielsweise pH-Wert, Sauerstoffpartialdruck, Druck, Dehnung, Beschleunigung, Proteinkonzentration und/oder Spannung. Sensoren, die als solche bekannt sind und für die Integration in das Grundbauteil 33 geeignet sind, kann der Fachmann je nach auszuführender Meßanwendung auswählen. Mit Hilfe der Sensoreinrichtung 35 und der Telemetrieeinrichtung 34 ist eine telemetrische Meßsensoreinheit gebildet. Bei der Sensoreinrichtung 35 handelt es sich beispielsweise um einen Meßchip, einen Thermistor, einen ASIC oder dergleichen.

Die telemetrische Meßsensoreinheit kann als aktives Modul gebildet sein, welches über eine eigenen Energieversorgung verfügt, eine Batterie oder/und einen Akkumulator, die/der zumindest zeitweise die notwendige Energie zur Verfügung stellt. Sie kann auch als passives Modul ausgeführt sein, bei dem die benötigte Energie für die Sensoreinrichtung 35 und die Telemetrieeinrichtung 34 bedarfsweise von außen über eine kabellose Verbindung eingekoppelt wird.

Die in der Sensoreinrichtung 35 erzeugten Meßdaten für die Meßgröße(n) werden von der Sensoreinrichtung 35 über die Datenübertragungsverbindung 36 an die Telemetrieeinrichtung 34 übertragen. Die Telemetrieeinrichtung 34 verfügt über geeignete Sende-/Empfangsmittel, um die Meßdaten auf Anfrage kabellos an eine Auswerteeinrichtung (nicht dargestellt) zu übertragen. Die Datenübertragung kann mit Hilfe Auslesens der Daten ausgeführt werden, wozu ein externes Lesesystem (nicht dargestellt) genutzt wird. Das Lesesystem dient dazu, die Daten aus der Sensoreinrichtung 35 direkt oder über die Telemetrieeinrichtung 34 auf kabellosem, telemetrischem Weg auszulesen und gegebenenfalls einem sogenannten "Datalogger" oder einer Auswerteeinheit zuzuführen. Es kann vorgesehen sein, daß das Lesesystem in der. Lage ist, eine Energieversorgung für die telemetrische Meßsensoreinheit bereitzustellen. Im Fall einer eigenen Energieversorgung der telemetrische Meßsensoreinheit, beispielsweise in Form einer Batterie oder eines Akkumulators, kann diese mit Hilfe des Lesesystems über eine kabellose Verbindung aufgeladen werden.

Die elektronischen Komponenten des Systems umfassen einen Signalkonditionierungschip (z. B. ASIC) mit AD-Wandler und Filterung, eine digitale Schnittstelle zur Rechnerankopplung, einem Transponder-IC mit integriertem Prozessor, ein Speichermedium, eine Echtzeituhr, eine Antennenspule und gegebenenfalls eine Energiequelle mit Powermanagement. Der Signalkonditionierungschip/ASIC ermöglicht eine elektronische Korrektur der individuellen Sensorfehler und gestattet somit eine hochpräzise Messung der Vitalparameter.

Das Gehäuse für die Telemetrie-Einheit besteht aus einem biokompatiblen polymeren Werkstoff, mit und ohne Beschichtung. Die Form und die technische Ausführung ist abhängig vom Einsatzort bzw. der Anwendung. Im Rahmen des Powermanagements kann ein Sleep-Modus aktiviert werden zur Reduzierung des Energieverbrauchs oder bei fehlender Energieversorgung.

Auf diese Weise ist es möglich, nach dem Anordnen des Bauteils 30 in einem Implantat, beispielsweise dem in Figur 1 gezeigten Implantat 2, und der Implantation des Implantats im Körper eines Lebewesens Meßdaten zu erfassen und zu einer Auswerteeinrichtung außerhalb des Körpers des Lebewesens zu übertragen. Die Übertragung derartiger Meßdaten ist als solche in Verbindung mit Sensoren im Körper eines Lebewesens bekannt und wird hier deshalb nicht weiter erläutert.

Gemäß Figur 3 ist an dem Basisteil 32 ein Gewindeabschnitt 37 vorgesehen, der dazu dient, daß Bauteil 30 bei der Montage an dem Implantat in die Implantatausnehmung einzuschrauben.

Figur 4 zeigt einen Querschnitt eines weiteren Bauteils 40, welches beispielsweise als eine Ausführungsform für das Bauteil 6 in der Anordnung nach Figur 1 verwendet werden kann. Im Grundbauteil 41 des Bauteils 40 sind eine Telemetrieeinrichtung 42 sowie eine Sensoreinrichtung 43 vorgesehen. Zur Übertragung von mit Hilfe der Sensoreinrichtung 43 erzeugter Meßdaten an die Telemetrieeinrichtung 42 ist wiederum eine Datenübertragungsverbindung 44 vorgesehen. Im Unterschied zu der Ausführungsform nach Figur 3 verfügt das Bauteil 40 im Bereich des Basisteils 45 nicht über einen Gewindeabschnitt. Es ist vielmehr ein verbreitertes Fußteil 46 vorgesehen. Wird das Bauteil 40 beim Anordnen in einer Implantatausnehmung mit dem Fußteil 46 zuerst in die Implantatausnehmung eingerührt, so drücken überstehende Seitenabschnitte 47 des Fußteil 46 gegen die Wandung der Implantatausnehmung, wodurch eine Klemmwirkung zum Befestigen des Bauteils 40 an dem Implantat entfaltet wird. Hierbei kann vorgesehen sein, daß das Grundbauteil 41 aus einem in gewissem Umfang nachgebenden Material gebildet ist, so daß die überstehenden Seitenabschnitte 47 beim Einführen in die Implantatausnehmung zum Teil eingedrückt werden.

In Abhängigkeit von den Anwendungsfällen kann der Fachmann zwischen unterschiedlichen Materialien für die Grundbauteile 41 bzw. 33 auswählen. Während bei der Ausführungsform des Bauteils 30 ein eher festes Material zu nutzen ist, beispielsweise ein Metall, um den Gewindeabschnitt 37 auszubilden, kann für die Ausführungsform des Bauteils 40 ein Material gewählt werden, das in gewissem Umfang elastisch oder nicht elastisch verformbar ist, beispielsweise ein Kunststoff. Wenn das Bauteil 40 zum Teil durch eine als Durchbruch ausgeführte Implantatausnehmung hindurch gesteckt wird, verhindert das Fußteil ein Herausrutschen des Bauteils 40.

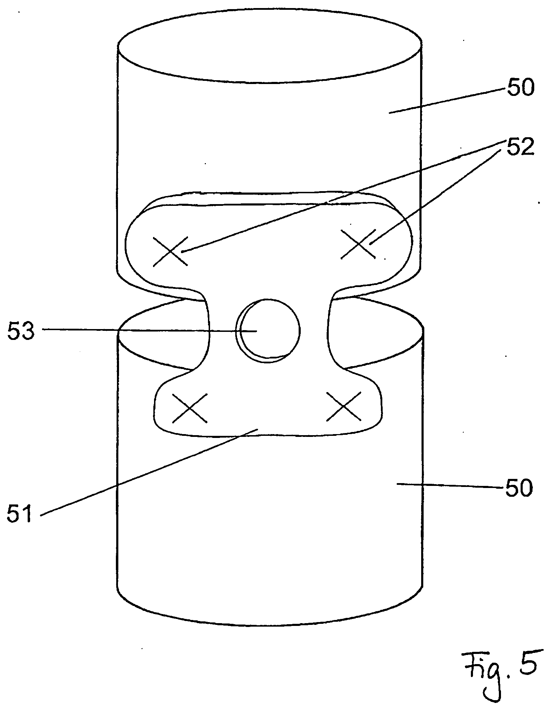

Figur 5 zeigt einen Wirbelkörper 50, an dem ein Implantat 51 mittels Schrauben 52 befestigt ist. Eine Öffnung 53 in dem Implantat 51 ist ungenutzt, so daß in dieser Öffnung 53 ein Bauteil angeordnet werden kann, wie es beispielhaft unter Bezugnahme auf die Figuren 3 und 4 beschrieben wurde.

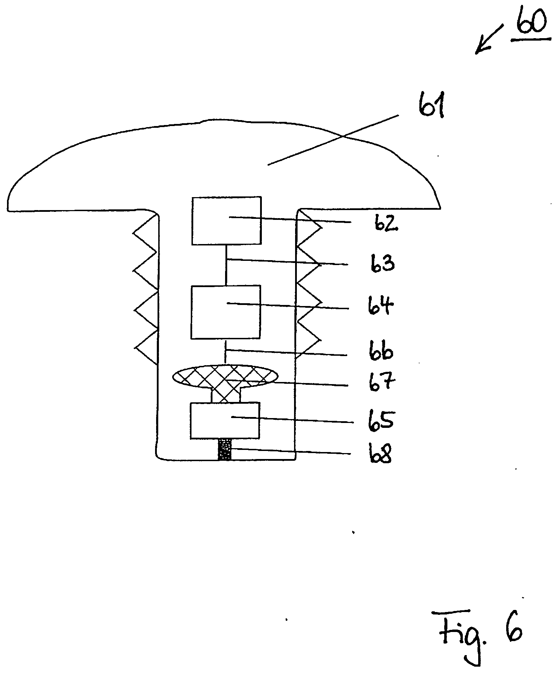

Figur 6 zeigt ein anderes Bauteil 60 im Querschnitt, welches insbesondere als eine Ausführungsform für das Bauteil 6 in der Anordnung nach Figur 1 verwendet werden kann. In eine Kapselung 61 ist eine Applikationseinheit 62 integriert. Die Applikationseinheit 62 empfängt mit Hilfe einer Sensoreinheit (nicht dargestellt) erfaßte Meßsignale über eine drahtlose oder drahtgebundene Verbindung zur elektronischen Datenübertragung. Über eine Signalleitung 63 ist die Applikationseinheit 62 mit einer Steuereinheit 64 verbunden. In der Steuereinheit 64 sind in Form elektronischer Daten typische Kennlinien für die mit Hilfe der Sensoreinheit zu erfassenden Meßsignale in einem Speicher gespeichert. Die typischen Kennlinien können von außen über eine Telemetrie-Anbindung der Sensoreinheit durch geänderte Kennlinien ersetzt werden. In der Steuereinheit 64 werden zum Ansteuern einer Pumpeinrichtung 65, die über eine Steuerleitung 66 mit der Steuereinheit 64 verbunden ist, Steuersignale auf Basis eines Istwert/Sollwert-Vergleiches der erfaßten Meßsignale mit dem für die typischen Kennlinien gespeicherten Daten erzeugt. In Abhängigkeit von den Steuersignalen wird dann mit Hilfe der Pumpeinrichtung 65 ein Wirkstoff aus einem Wirkstoffvorrat 67 über eine verschließbare Öffnung 68 abgegeben. Der Wirkstoff ist eine chemische Verbindung beliebiger Art, die zur Erzielung einer medizinischen Wirkung an die Umgebung des anderen Bauteils 60 abgegeben wird, beispielsweise um einen Heilungsprozeß zu beeinflußen. Aufgrund der Wirkstoffabgabe kann eine Änderung der erfaßten Meßsignale an der Sensoreinheit erwartet werden. Die geänderten Meßsignale an der Sensoreinheit werden nach Übertragung an die Applikationseinheit 62 dann wiederum in der Steuereinheit 64 mit Sollwerten verglichen, so daß eine Art Regelkreis für die Wirkstoffabgabe gebildet ist. Der Fachmann kann zur Implementierung des anderen Bauteils 60 nach Figur 6 geeignete Mikroprozessortechnik sowie Mikrosystemtechnik, beispeilsweise Mikropumptechnik auswählen, die in verschiedenen Formen bekannt ist.

Die Wirkstofffreigabe kann auch passiv erfolgen, zum Beispiel mittels eines bioabbaubarem (Matrix)systems oder der Freigabe durch Diffusion aus einer Matrix.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. System, umfassend
- ein Stützimplantat (2; 51), insbesondere Platte oder Schiene aus einem Material mit hoher Steifigkeit, oder ein Ersatzimplantat, insbesondere künstliches Hüft-, Knieoder Schultergelenk, mit mehreren, beim Implantieren nutzbaren Implantatausnehmungen (4; 53) zum Aufnehmen von Implantat-Befestigungsmitteln (5; 52), und
- ein Bauteil (6; 30; 40; 60) zum Anordnen an dem Stützimplantat (2; 51) oder Ersatzimplantat, mit:
- einem Grundbauteil (33; 41);
- mindestens einer in dem Grundbauteil (33; 41) angeordneten Sensoreinrichtung (35; 43) zum Erfassen einer Messgröße und zum Erzeugen von Messdaten für die erfasste Messgröße;
- einer in dem Grundbauteil (33; 41) angeordneten Telemetrieeinrichtung (34; 42) zum Senden und/oder Empfangen von Daten; und
- einer Datenübertragungsverbindung (36; 44) zwischen der mindestens einen Sensoreinrichtung (35; 43) und der Telemetrieeinrichtung (34; 42) zum Übertragen von Daten zwischen der mindestens einen Sensoreinrichtung (35; 43) und der Telemetrieeinrichtung (34; 42), wobei die Daten die Messdaten umfassen;
wobei an dem Grundbauteil (33; 41) ein Aufnahmeraum (67) zum Aufnehmen eines Wirkstoffs gebildet ist, wobei der Aufnahmeraum (67) mit einer Öffnung (68) zum Abgeben des Wirkstoffs nach außen in Verbindung steht, und
wobei an dem Grundbauteil (33; 41) Montagemittel (37; 47) zum lösbaren Montieren des Grundbauteils (33; 41) in einer der Implantatausnehmungen (4; 53) des Stützimplantats (2; 51) oder Ersatzimplantats gebildet sind.

2. System nach Anspruch 1, **dadurch gekennzeichnet, dass** die Montagemittel einen Montageabschnitt (37; 47) zum wenigstens teilweisen Einführen in die Implantatausnehmung (4; 53) umfassen.

3. System nach Anspruch 2, **dadurch gekennzeichnet, dass** die Implantatausnehmung (4; 53) einen Innengewindeabschnitt aufweist und im Bereich des - Montageabschnitts eine Gewindeabschnitt (37) zum Einschrauben des Grundbauteils (33) in die Implantatausnehmung (4; 53) gebildet ist.

4. System nach einem der vorangehenden Ansprüche, **dadurch gekennzeichnet, dass** das Grundbauteil (33; 41) im Längsschnitt einen im wesentlichen T-förmigen Querschnitt mit einem Kopfteil (6a) und einem Basisteil (6b) aufweist.

5. System nach einem der vorangehenden Ansprüche, **dadurch gekennzeichnet, dass** die mindestens eine Sensoreinrichtung (35; 43) im Bereich eines Endabschnitts des Grundbauteils (33; 41) und die Telemetrieeinrichtung (34; 42) im Bereich eines gegenüberliegenden Endabschnitts des Grundbauteils (33; 41) angeordnet sind.

6. System nach Anspruch 4 oder 5, **dadurch gekennzeichnet, dass** die Telemetrieeinrichtung (34; 42) im Wesentlichen in dem Kopfteil (6a) des Grundbauteils (33; 41) angeordnet ist.

7. System nach einem der Ansprüche 1 bis 6, **dadurch gekennzeichnet, dass** eine Abgabeeinrichtung zum kontrollierten Abgeben des Wirkstoffs aus dem Aufnahmeraum (67) durch die Öffnung (68) vorgesehen ist.

8. System nach Anspruch 7, **dadurch gekennzeichnet, dass** die Abgabeeinrichtung eine Pumpeinrichtung (65) zum Pumpen einer Menge des Wirkstoffs aus dem Aufnahmeraum (67) durch die Öffnung (68) umfasst.

9. System nach Anspruch 7 oder 8, **dadurch gekennzeichnet, dass** die Abgabeeinrichtung einen Öffnungsmechanismus zum Öffnen/Schließen der Öffnung (68) umfasst.

10. System nach einem der Ansprüche 7 bis 9, **dadurch gekennzeichnet, dass** die Abgabeeinrichtung über einer weitere Datenübertragungsverbindung (63) zum - Übertragen von Daten mit der Telemetrieeinrichtung (34; 42) verbunden ist.

11. System nach einem der Ansprüche 7 bis 10, **gekennzeichnet durch** eine Steuereinheit (64), die mit der mindestens einen Sensoreinrichtung und der Abgabeeinrichtung verbunden ist, um die Erfassung der Messdaten mit Hilfe der mindestens einen Sensoreinrichtung und die Abgabe des Wirkstoffs mit Hilfe der Abgabeeinrichtung gemeinsam zu steuern.

12. Bauteil zur Verwendung in einem System nach einem der Ansprüche 1 bis 11, mit:
- einem Grundbauteil (33; 41);
- mindestens einer in dem Grundbauteil (33; 41) angeordneten Sensoreinrichtung (35; 43) zum Erfassen einer Messgröße und zum Erzeugen von Messdaten für die erfasste Messgröße;
- einer in dem Grundbauteil (33; 41) angeordneten Telemetrieeinrichtung (34; 42) zum Senden und/oder Empfangen von Daten; und
- einer Datenübertragungsverbindung (36; 44) zwischen der mindestens einen Sensoreinrichtung (35; 43) und der Telemetrieeinrichtung (34; 42) zum Übertragen von Daten zwischen der mindestens einen Sensoreinrichtung (35; 43) und der Telemetrieeinrichtung (34; 42), wobei die Daten die Messdaten umfassen;
wobei an dem Grundbauteil (33; 41) ein Aufnahmeraum (67) zum Aufnehmen eines Wirkstoffs gebildet ist, wobei der Aufnahmeraum (67) mit einer Öffnung (68) zum Abgeben des Wirkstoffs nach außen in Verbindung steht,
wobei an dem Grundbauteil (33; 41) Montagemittel (37; 47) zum lösbaren Montieren des Grundbauteils (33; 41) in einer Implantatausnehmung (4; 53) eines Implantats (2; 51) gebildet sind, und
wobei das Grundbauteil (33; 41) im Längsschnitt einen im Wesentlichen T-förmigen oder pilzförmigen Querschnitt mit einem Kopfteil (6a) und einem Basisteil (6b) aufweist.

13. Bauteil nach Anspruch 12, **dadurch gekennzeichnet, dass** die Montagemittel einen Montageabschnitt (37; 47) zum wenigstens teilweisen Einführen in die Implantatausnehmung (4; 53) umfassen, wobei im Bereich des Montageabschnitts vorzugsweise ein Gewindeabschnitt (37) zum Einschrauben des Grundbauteils (33) in die Implantatausnehmung (4; 53) gebildet ist.

14. Bauteil nach Anspruch 12 oder 13, **dadurch gekennzeichnet, dass** die mindestens eine Sensoreinrichtung (35; 43) im Bereich eines Endabschnitts des Grundbauteils (33; 41) und die Telemetrieeinrichtung (34; 42) im Bereich eines gegenüberliegenden Endabschnitts des Grundbauteils (33; 41) angeordnet sind.

15. Bauteil nach einem der Ansprüche 12 bis 14, **dadurch gekennzeichnet, dass** die Telemetrieeinrichtung (34; 42) im Wesentlichen in dem Kopfteil (6a) des Grundbauteils (33; 41) angeordnet ist.

16. Bauteil nach einem der Ansprüche 12 bis 15, **dadurch gekennzeichnet, dass** eine Abgabeeinrichtung zum kontrollierten Abgeben des Wirkstoffs aus dem Aufnahmeraum (67) durch die Öffnung (68) vorgesehen ist.

17. Bauteil nach Anspruch 16, **dadurch gekennzeichnet, dass** die Abgabeeinrichtung eine Pumpeinrichtung (65) zum Pumpen einer Menge des Wirkstoffs aus dem Aufnahmeraum (67) durch die Öffnung (68) umfasst.

18. Bauteil nach Anspruch 16 oder 17, **dadurch gekennzeichnet, dass** die Abgabeeinrichtung einen Öffnungsmechanismus zum Öffnen/Schließen der Öffnung (68) umfasst.

19. Bauteil nach einem der Ansprüche 16 bis 18, **dadurch gekennzeichnet, dass** die Abgabeeinrichtung über einer weitere Datenübertragungsverbindung (63) zum Übertragen von Daten mit der Telemetrieeinrichtung (34; 42) verbunden ist.

20. Bauteil nach einem der Ansprüche 16 bis 19, **gekennzeichnet durch** eine Steuereinheit (64), die mit der mindestens einen Sensoreinrichtung und der Abgabeeinrichtung verbunden ist, um die Erfassung der Messdaten mit Hilfe der mindestens einen Sensoreinrichtung und die Abgabe des Wirkstoffs mit Hilfe der Abgabeeinrichtung gemeinsam zu steuern.

## Claims

1. A system comprising:
- a support implant (2; 1), in particular a plate or splint of a material of high stiffness, or a replacement implant, in particular an artificial hip joint, knee joint or shoulder joint, with several implant recesses (4; 53) usable upon implanting for receiving implant fixation means (5; 52), and
- a component (6; 30; 40; 60) for arrangement on the support implant (2; 51) or replacement implant, with:
- a base component (33; 41);
- at least one sensor device (35; 43) arranged in the base component (33; 41) for capturing a measurand and for producing measuring data for the captured measurand;
- a telemetric device (34; 42) arranged in the base component (33; 41) for sending and/or receiving data; and
- a data transmission connection (36; 44) between the at least one sensor device (35; 43) and the telemetric device (34; 42) for transmitting data between the at least one sensor device (35; 43) and the telemetric device (34; 42), wherein the data comprise the measuring data;
wherein on the base component (33; 41) a receiving space (67) is formed for receiving an active substance, wherein the receiving space (67) is connected with an opening (68) for dispensing the active substance toward the outside, and
wherein on the base component (33; 41) mounting means (37; 47) are formed for detachably mounting the base component (33; 41) in one of the implant recesses (4; 53) of the support implant (2; 51) or replacement implant.

2. The system according to claim 1, **characterized in that** the mounting means comprise a mounting section (37; 47) for at least partial insertion into the implant recess (4; 53).

3. The system according to claim 2, **characterized in that** the implant recess (4; 53) has an inner threaded section and in the area of the mounting section a threaded section (37) is formed for screwing the base component (33) into the implant recess (4; 53).

4. The system according to any of the preceding claims, **characterized in that** the base component (33; 41) in longitudinal section has a substantially T-shaped cross section with a head portion (6a) and a base portion (6b).

5. The system according to any of the preceding claims, **characterized in that** the at least one sensor device (35; 43) is arranged in the area of an end section of the base component (33; 41) and the telemetric device (34; 42) is arranged in the area of an end section of the base component (33; 41) that lies opposite thereto.

6. The system according to claim 4 or 5, **characterized in that** the telemetric device (34; 42) is arranged substantially in the head portion (6a) of the base component (33; 41).

7. The system according to any of the claims 1 to 6, **characterized in that** a dispensing device is provided for the controlled dispensing of the active substance from the receiving space (67) through the opening (68).

8. The system according to claim 7, **characterized in that** the dispensing device comprises a pumping device (65) for pumping an amount of the active substance from the receiving space (67) through the opening (68).

9. The system according to claim 7 or 8, **characterized in that** the dispensing device comprises an opening mechanism for opening/ closing the opening (68).

10. The system according to any of the claims 7 to 9, **characterized in that** the dispensing device is connected to the telemetric device (34; 42) via a further data transmission connection (63) for transmitting data.

11. The system according to any of the claims 7 to 10, **characterized by** a control unit (64) connected to the at least one sensor device and the dispensing device, in order to jointly control the capture of the measuring data with the aid of the at least one sensor device and the dispensing of the active substance with the aid of the dispensing device.

12. A component for use in a system according to any of the claims 1 to 11, with:
- a base component (33, 41);
- at least one sensor device (35; 43) arranged in the base component (33; 41) for capturing a measurand and for producing measuring data for the captured measurand;
- a telemetric device (34; 42) arranged in the base component (33, 41) for sending and/ or receiving data; and
- a data transmission connection (36; 44) between the at least one sensor device (35; 43) and the telemetric device (34; 42) for transmitting data between the at least one sensor device (35; 43) and the telemetric device (34; 42), wherein the data comprise the measuring data;
wherein on the base component (33; 41) a receiving space (67) is formed for receiving an active substance, wherein the receiving space (67) is connected with an opening (68) for dispensing the active substance toward the outside,
wherein on the base component (33; 41) mounting means (37; 47) are formed for detachably mounting the base component (33; 41) in an implant recess (4; 53) of an implant (2; 51), and
wherein the base component (33; 41) in longitudinal section has a substantially T-shaped or mushroom-shaped cross-section with a head portion (6a) and a base portion (6b).

13. The component according to claim 12, **characterized in that** the mounting means comprise a mounting section (37; 47) for at least partial insertion into the implant recess (4; 53), wherein in the area of the mounting section there is preferably formed a threaded section (37) for screwing the base component (33) into the implant recess (4; 53).

14. The component according to claim 12 or 13, **characterized in that** the at least one sensor device (35; 43) is arranged in the area of an end section of the base component (33; 41) and the telemetric device (34; 42) is arranged in the area of an end section of the base component (33; 41) lying opposite thereto.

15. The component according to any of the claims 12 to 14, **characterized in that** the telemetric device (34; 42) is arranged substantially in the head portion (6a) of the base component (33; 41).

16. The component according to any of the claims 12 to 15, **characterized in that** a dispensing device is provided for the controlled dispensing of the active substance from the receiving space (67) through the opening (68).

17. The component according to claim 16, **characterized in that** the dispensing device comprises a pumping device (65) for pumping an amount of the active substance from the receiving space (67) through the opening (68).

18. The component according to claim 16 or 17, **characterized in that** the dispensing device comprises an opening mechanism for opening/ closing the opening (68).

19. The component according to any of the claims 16 to 18, **characterized in that** the dispensing device is connected to the telemetric device (34; 42) via a further data transmission connection (63) for transmitting data.

20. The component according to any of the claims 16 to 19, **characterized by** a control unit (64) connected to the at least one sensor device and the dispensing device, in order to jointly control the capture of the measuring data with the aid of the at least one sensor device and the dispensing of the active substance with the aid of the dispensing device.

## Revendications

1. Système, comprenant
- un implant de soutien (2; 51), en particulier une plaque ou broche en un matériau à haute rigidité, ou un implant de remplacement, en particulier articulation artificielle de la hanche, du genou ou de l'épaule, comportant plusieurs évidements d'implants (4; 53) utilisables lors de l'implantation et destinées au logement des moyens de fixation d'implants (5; 52), et
- un composant (6; 30; 40; 60) destiné à l'agencement sur l'implant de soutien (2; 51) ou implant de remplacement, comportant :
- un composant de base (33; 41);
- au moins un dispositif de capteur (35; 43) agencé dans le composant de base (33; 41) et destiné à la saisie d'une grandeur de mesure et à la génération de données de mesure pour la grandeur de mesure saisie;
- un dispositif de télémétrie (34; 42) agencé dans le composant de base (33; 41) et destiné à l'envoi et/ou à la réception de données; et
- une liaison de transmission de données (36; 44) entre le au moins un dispositif de capteur (35; 43) et le dispositif de télémétrie (34; 42) pour la transmission de données entre le au moins un dispositif de capteur (35; 43) et le dispositif de télémétrie (34; 42), les données comprenant les données de mesure;
cependant que, au composant de base (33; 41), un espace de logement (67) destiné au logement d'une substance active est constitué, l'espace de logement (67) étant en liaison avec une ouverture (68) destinée à la délivrance de la substance active vers l'extérieur, et
cependant que, au composant de base (33; 41), des moyens de montage (37; 47) destinés au montage amovible du composant de base (33; 41) sont constitués dans un des évidements d'implants (4; 53) de l'implant de soutien (2; 51) ou implant de remplacement.

2. Système selon la revendication 1, **caractérisé en ce que** les moyens de montage comprennent un tronçon de montage (37; 47) destiné à l'introduction au moins partielle dans l'évidement d'implant (4; 53).

3. Système selon la revendication 2, **caractérisé en ce que** l'évidement d'implant (4; 53) comporte un tronçon à filetage intérieur et **en ce que**, dans la zone du tronçon de montage, un tronçon à filetage (37) destiné au vissage du composant de base (33) dans l'évidement d'implant (4; 53) est constitué.

4. Système selon une des revendications précédentes, **caractérisé en ce que** le composant de base (33; 41) présente, en coupe longitudinale, une coupe transversale essentiellement en en T ayant une partie de tête (6a) et une partie de base (6b).

5. Système selon une des revendications précédentes, **caractérisé en ce que** le au moins un dispositif de capteur (35; 43) est agencé dans la zone d'un tronçon d'extrémité du composant de base (33; 41) et le dispositif de télémétrie (34; 42) est agencé dans la zone d'un tronçon d'extrémité opposé du composant de base (33; 41).

6. Système selon la revendication 4 ou 5, **caractérisé en ce que** le dispositif de télémétrie (34; 42) est agencé essentiellement dans la partie de tête (6a) du composant de base (33; 41).

7. Système selon une des revendications de 1 à 6, **caractérisé en ce qu'**un dispositif de délivrance destiné à la délivrance contrôlée de la substance active depuis l'espace de logement (67) à travers l'ouverture (68) est prévu.

8. Système selon la revendication 7, **caractérisé en ce que** le dispositif de délivrance comprend un dispositif de pompage (65) destiné au pompage d'une quantité de la substance active depuis l'espace de logement (67) à travers l'ouverture (68).

9. Système selon la revendication 7 ou 8, **caractérisé en ce que** le dispositif de délivrance comprend un mécanisme d'ouverture destiné à ouvrir/fermer l'ouverture (68).

10. Système selon une des revendications de 7 à 9, **caractérisé en ce que** le dispositif de délivrance est, par l'intermédiaire d'une liaison supplémentaire de transmission de données (63), relié avec le dispositif de télémétrie (34; 42) pour la transmission de données.

11. Système selon une des revendications de 7 à 10, **caractérisé par** une unité de commande (64) qui est reliée avec le au moins un dispositif de capteur et avec le dispositif de délivrance afin de commander conjointement la saisie des données de mesure à l'aide du au moins un dispositif de capteur et la délivrance de la substance active à l'aide du dispositif de délivrance.

12. Composant destiné à l'utilisation dans un système selon une des revendications de 1 à 11, comportant:
- un composant de base (33; 41);
- au moins un dispositif de capteur (35; 43) agencé dans le composant de base (33; 41) et destiné à la saisie d'une grandeur de mesure et à générer des données de mesure pour la grandeur de mesure saisie;
- un dispositif de télémétrie (34; 42) agencé dans le composant de base (33; 41) et destiné à l'envoi et/ou à la réception de données; et
- une liaison de transmission de données (36; 44) entre le au moins un dispositif de capteur (35; 43) et le dispositif de télémétrie (34; 42) pour la transmission de données entre le au moins un dispositif de capteur (35; 43) et le dispositif de télémétrie (34; 42), les données comprenant les données de mesure;
cependant que, au composant de base (33; 41), un espace de logement (67) destiné au logement d'une substance active est constitué, l'espace de logement (67) étant en liaison avec une ouverture (68) pour délivrer la substance active vers l'extérieur,
cependant que, au composant de base (33; 41), des moyens de montage (37; 47) destinés au montage amovible du composant de base (33; 41) sont constitués dans un évidement d'implant (4; 53) d'un implant (2; 51), et
cependant que le composant de base (33; 41) présente, en coupe longitudinale, une coupe transversale essentiellement en T ou fongiforme ayant une partie de tête (6a) et une partie de base (6b).

13. Composant selon la revendication 12, **caractérisé en ce que** les moyens de montage comprennent
un tronçon de montage (37; 47) destiné à l'introduction au moins partielle dans l'évidement d'implant (4; 53), cependant que, dans la zone du tronçon de montage, de préférence un tronçon à filetage (37) destiné au vissage du composant de base (33) dans l'évidement d'implant (4; 53) est constitué.

14. Composant selon la revendication 12 ou 13, **caractérisé en ce que** le au moins un dispositif de capteur (35; 43) est agencé dans la zone d'un tronçon d'extrémité du composant de base (33; 41) et le dispositif de télémétrie (34; 42) est agencé dans la zone d'un tronçon d'extrémité opposé du composant de base (33; 41).

15. Composant selon une des revendications de 12 à 14, **caractérisé en ce que** le dispositif de télémétrie (34; 42) est agencé essentiellement dans la partie de tête (6a) du composant de base (33; 41).

16. Composant selon une des revendications de 12 à 15, **caractérisé en ce qu'**un dispositif de délivrance destiné à la délivrance contrôlée de la substance active depuis l'espace de logement (67) à travers l'ouverture (68) est prévu.

17. Composant selon la revendication 16, **caractérisé en ce que** le dispositif de délivrance comprend un dispositif de pompage (65) destiné au pompage d'une quantité de la substance active depuis l'espace de logement (67) à travers l'ouverture (68).

18. Composant selon la revendication 16 ou 17, **caractérisé en ce que** le dispositif de délivrance comprend un mécanisme d'ouverture destiné à l'ouverture/la fermeture de l'ouverture (68).

19. Composant selon une des revendications de 16 à 18, **caractérisé en ce que** le dispositif de délivrance est, par l'intermédiaire d'une liaison supplémentaire de transmission de données (63), relié avec le dispositif de télémétrie (34; 42) pour la transmission de données.

20. Composant selon une des revendications de 16 à 19, **caractérisé par** une unité de commande (64) qui est reliée avec le au moins un dispositif de capteur et avec le dispositif de délivrance afin de commander conjointement la saisie des données de mesure à l'aide du au moins un dispositif de capteur et la délivrance de la substance active à l'aide du dispositif de délivrance.
